# EUROPEAN PATENT APPLICATION

(11) **EP 4 282 884 A1**
(43) Date of publication of application: **29.11.2023**
(21) Application number: 22755554.7
(22) Date of filing: 17.02.2022
(51) Int. Cl.: C07K 19/00, C07K 14/71, C07K 14/495, A61K 39/395, C12N 15/62, C12N 15/63, A61P 35/00

(54) **MULTI-DOMAIN FUSION PROTEIN AND USE THEREOF**

(30) Priority: 22.02.2021 WO PCT/CN2021/077282
(71) Applicant: Zhejiang Doer Biologics Co., Ltd., Hangzhou, Zhejiang 310018 (CN)
(72) Inventor: HUANG, Yanshan, Hangzhou, Zhejiang 310018 (CN)
(74) Representative: Canzler & Bergmeier Patentanwälte Partnerschaft mbB
(86) International application number: PCT/CN2022/076610
(87) International publication number: WO 2022/174781

(57) **Abstract**

Provided in the present invention is a fusion protein, which comprises an anti-PD-L1 single-domain antibody fragment, an antagonistic VEGF fragment and a TGF-β binding fragment. The multi-domain fusion protein with an anticancer activity provided in the present invention can organically combine the functions of blocking PD-L1/PD-1 interaction with an anti-PD-L1 monoclonal antibody, reducing microvascular growth and inhibiting metastatic diseases with an anti-VEGF monoclonal antibody, and relieving TGF-β signal tolerance of cancer cells and enhancing immune response with a TGF-β receptor in an antibody fusion protein molecule, so that the multi-domain fusion protein can be used for treating tumors.

## Description

### FIELD OF TECHNOLOGY

The present disclosure relates to the field of biotechnology, and in particular to a multi-domain fusion protein with anticancer activity, preparation method, and use thereof.

### BACKGROUND

Tumor cells differ from normal cells in their high metabolic requirements for growth. Tumor cells rely on nutrients and oxygen provided by blood vessels, process metabolites through blood vessels, and promote the formation of new blood vessels based on the existing vessels. Among the pro-angiogenic factors secreted by tumors, human vascular endothelial growth factor (VEGF), especially VEGF-A, is an important factor contributing to tumor angiogenesis (Josep Garcia et al., 2020, Cancer Treatment Reviews 86:1-2). Therefore, inhibition of the VEGF signaling pathway can limit the progression of a variety of tumors. For example, Bevacizumab (AVASTIN^{®}), a humanized anti-VEGF monoclonal antibody, can bind to VEGF to prevent VEGF from interacting with VEGF receptors (Flt1 and KDR) on the surface of an endothelial cell.

Bevacizumab is currently approved by the FDA for the treatment of metastatic colorectal cancer, advanced, metastatic, or recurrent non-small cell lung cancer, and recurrent glioblastoma.

Transforming growth factor TGF-β is a cytokine that maintains tissue homeostasis by regulating cell growth, differentiation, proliferation, and survival. Although the TGF-β pathway can control tumors by promoting cell-cycle arrest and cell apoptosis in the early stage, in the late stages of tumors, TGF-β allows tumors to escape by inhibiting cytotoxic T cells and promoting cancer cells' proliferation, invasion, and metastasis. This functional transformation is known as the "TGF-β paradox". The TGF-β signal pathway induces T_{H}1 cells to differentiate into Tregs, impairs the activation of CD8+ effector T cells, and limits the development of central memory T cells, which fundamentally affect the function of tumor-infiltrating T cells. In mammals, there are three main subtypes of TGF-β, which are TGF-β1, TGF-β2, and TGF-β3, respectively. Tumors are protected from immune surveillance through high-level TGF-β expression. In addition, high-level TGF-β expression in non-small cell lung cancer (NSCLC), renal cell carcinoma (CRC), gastric cancer, and prostate cancer is associated with tumor progression and poor prognosis (Marin-Acevedo et al., Journal of Hematology & Oncology, 11:39, 2018). This suggests that TGF-β antagonism will be a potential new direction for tumor therapy.

At present, anti-PD-1/PD-L1 monoclonal antibodies, which serve as immune checkpoint inhibitors, are widely used in oncology treatment. M7824, a fusion of the anti-PD-L1 monoclonal antibody Avelumab with TGF-β receptor II (TGF-β Trap), has entered the clinical phase. However, a combination of an anti-PD-1/PD-L1 monoclonal antibody, a TGF-β antagonist, and an anti-VEGF monoclonal antibody has not been reported yet.

### SUMMARY

In view of the above-described shortcomings of the prior art, the present disclosure provides a multi-domain fusion protein with anticancer activity, preparation method, and use thereof.

For these and other related purposes, the present disclosure provides a fusion protein, including an anti-PD-L1 single domain antibody fragment, an anti-VEGF fragment, and a TGF-β binding fragment.

The present disclosure provides an isolated polynucleotide encoding the fusion protein described above.

The present disclosure provides a construct, including the isolated polynucleotide as described above.

The present disclosure provides an expression system, including the construct or the isolated polynucleotide which is incorporated into a genome.

The present disclosure provides a method of preparing the fusion protein, including: culturing the expression system as described above under a condition suitable for expressing the fusion protein, and performing isolation and purification to provide the fusion protein.

The present disclosure provides a use of the above fusion protein, or a culture of the above expression system in the preparation of medications.

The present disclosure provides a pharmaceutical composition, including the above fusion protein or a culture of the above expression system.

### BRIEF DESCRIPTION OF THE DRAWINGS

FIG. 1 shows concentration-time curves of TAF6 and Avastin.
FIG. 2 shows tumor inhibiting efficacy of multi-domain fusion protein in subcutaneous MDA-MB-231 breast cancer xenograft model using PBMC humanized mice.
FIG. 3 shows tumor inhibiting efficacy of multi-domain fusion protein in subcutaneous MDA-MB-231 breast cancer xenograft model using PBMC humanized mice.
FIG. 4 shows tumor inhibiting efficacy of multi-domain fusion proteins TAF-6, M7824 analogue, and Avastin in subcutaneous MDA-MB-231 breast cancer xenograft model using PBMC humanized mice.
FIG. 5 shows tumor inhibiting efficacy of multi-domain fusion protein in subcutaneous Calu-6 lung cancer xenograft model using PBMC humanized mice.
FIG. 6 shows tumor inhibiting efficacy of multi-domain fusion proteins TAF-6, M7824 analogue, and Avastin in subcutaneous Calu-6 lung cancer xenograft model using PBMC humanized mice.
FIG. 7 shows tumor inhibiting efficacy of multi-domain fusion protein in subcutaneous HCT116 colon cancer xenograft model using PBMC humanized mice.
FIG. 8 shows tumor inhibiting efficacy of multi-domain fusion protein in subcutaneous Huh-7 liver cancer xenograft model using PBMC humanized mice.
FIG. 9 shows tumor inhibiting efficacy of multi-domain fusion proteins TAF-6, M7824 analogue, and Avastin in subcutaneous Huh-7 liver cancer xenograft model using PBMC humanized mice.
FIG. 10 shows tumor inhibiting efficacy of multi-domain fusion protein in subcutaneous HT1080 sarcoma cancer xenograft model using PBMC humanized mice.

### DETAILED DESCRIPTION

After a lot of research, the inventors unexpectedly discovered a fusion protein molecule, which can combine the functions of an anti-PDL1 monoclonal antibody, an anti-VEGF monoclonal antibody, and a TGF-β receptor, where the anti-PDL1 monoclonal antibody blocks the PD-L1/PD-1 interaction, the anti-VEGF monoclonal antibody inhibits microvascular growth and tumor metastasis, and the TGF-β receptor alleviates the abnormal T cell functions caused by TGF-β in the tumor microenvironment and enhances immune response. The fusion protein molecule has an excellent tumor suppression effect.

A first aspect of the present disclosure provides a fusion protein including an anti-PD-L1 single domain antibody fragment, an anti-VEGF fragment, and a TGF-β binding fragment. In the above fusion protein, the anti-PD-L1 single domain antibody fragment can usually be used to block the PD-L1/PD-1 interaction and increase IFN-γ and/or IL-2 expression in T lymphocytes, thereby inhibiting tumor growth. The anti-VEGF fragment can often include an Fc fraction that binds to an FcRn receptor, thereby extending the half-life in vivo and killing cancer cells by binding to effector cells expressing the Fc receptor. The TGF-β binding fragment can enhance the killing function of tumor-infiltrating T cells against tumor cells by removing overexpressed TGF-β from the tumor microenvironment.

The fusion protein provided by the present disclosure may include the anti-PD-L1 single domain antibody fragment. The anti-PD-L1 single domain antibody fragment described above may typically be a peptide or protein fragment capable of specifically binding to PD-L1. In the anti-PDL1 single domain antibody fragment, the light chain of the antibody is usually missing, and only a fragment corresponding to the heavy chain variable region is present. The binding characteristics of the anti-PD-L1 single domain antibody fragment may usually be determined by its three complementarity determining regions (CDR), where the complementarity determining regions are interspersed with the framework regions (FR) that do not directly participate in a binding reaction. These CDRs can form loops that are spatially structured in close proximity to each other through the β-sheet structure formed by the FRs distributed among them, thus forming the antigen-binding site of the antibody. For example, the complementarity determining regions of the above anti-PD-L1 single domain antibody fragment may have CDR1, CDR2, and CDR3, where CDR1 has an amino acid sequence including one of SEQ ID NOs: 1-5, CDR2 has an amino acid sequence including one of SEQ ID NOs: 6-9, and CDR3 has an amino acid sequence including one of SEQ ID NOs: 10-15.

In one embodiment of the present disclosure, the complementarity determining regions of the anti-PD-L1 single domain antibody fragment includes CDR1-CDR3, where CDR1 has an amino acid sequence of SEQ ID NO: 1, CDR2 has an amino acid sequence of SEQ ID NO: 6, and CDR3 has an amino acid sequence of SEQ ID NO: 10.

In another embodiment of the present disclosure, the complementarity determining regions of the anti-PD-L1 single domain antibody fragment includes CDR1-CDR3, where CDR1 has an amino acid sequence of SEQ ID NO: 2, CDR2 has an amino acid sequence of SEQ ID NO: 7, and CDR3 has an amino acid sequence of SEQ ID NO: 11.

In another embodiment of the present disclosure, the complementarity determining regions of the anti-PD-L1 single domain antibody fragment includes CDR1-CDR3, where CDR1 has an amino acid sequence of SEQ ID NO: 3, CDR2 has an amino acid sequence of SEQ ID NO: 7, and CDR3 has an amino acid sequence of SEQ ID NO: 12.

In another embodiment of the present disclosure, the complementarity determining regions of the anti-PD-L1 single domain antibody fragment includes CDR1-CDR3, where CDR1 has an amino acid sequence of SEQ ID NO: 4, CDR2 has an amino acid sequence of SEQ ID NO: 8, and CDR3 has an amino acid sequence of SEQ ID NO: 13.

In another embodiment of the present disclosure, the complementarity determining regions of the anti-PD-L1 single domain antibody fragment includes CDR1-CDR3, where CDR1 has an amino acid sequence of SEQ ID NO: 2, CDR2 has an amino acid sequence of SEQ ID NO: 7, and CDR3 has an amino acid sequence of SEQ ID NO: 14.

In another embodiment of the present disclosure, the complementary determining regions of the anti-PD-L1 single domain antibody fragment includes CDR1-CDR3, where CDR1 has an amino acid sequence of SEQ ID NO: 5, CDR2 has an amino acid sequence of SEQ ID NO: 9, and CDR3 has an amino acid sequence of SEQ ID NO: 15.

The anti-PD-L1 single domain antibody fragment may further include a framework region (FR). As described above, the CDR regions may be interspersed with the FR regions, for example, the anti-PD-L1 single domain antibody fragment may include FR1, CDR1, FR2, CDR2, FR3, CDR3, and FR4 arranged in sequence from the N-terminus to the C-terminus. The framework regions FRs include FR1-FR4, where FR1 has an amino acid sequence of SEQ ID No: 49, FR2 has an amino acid sequence including one of SEQ ID Nos: 50-52, FR3 has an amino acid sequence including one of SEQ ID Nos: 53-55, and FR4 has an amino acid sequence of SEQ ID No: 56.

In a specific embodiment of the present disclosure, the framework regions FRs include FR1-FR4;

FR1 has an amino acid sequence of SEQ ID No: 49, FR2 has an amino acid sequence of SEQ ID No: 50, FR3 has an amino acid sequence of SEQ ID No: 53, and FR4 has an amino acid sequence of SEQ ID No: 56;

FR1 has an amino acid sequence of SEQ ID No: 49, FR2 has an amino acid sequence of SEQ ID No: 51, FR3 has an amino acid sequence of SEQ ID No: 54, and FR4 has an amino acid sequence of SEQ ID No: 56;

FR1 has an amino acid sequence of SEQ ID No: 49, FR2 has an amino acid sequence of SEQ ID No: 52, FR3 has an amino acid sequence of SEQ ID No: 54, and FR4 has an amino acid sequence of SEQ ID No: 56; or

FR1 has an amino acid sequence of SEQ ID No: 49, FR2 has an amino acid sequence of SEQ ID No: 52, FR3 has an amino acid sequence of SEQ ID No: 55, and FR4 has an amino acid sequence of SEQ ID No: 56.

In another embodiment of the present disclosure, the anti-PD-L1 single domain antibody fragment may include: a) a polypeptide fragment having an amino acid sequence selected from SEQ ID NOs:16-21; or b) a polypeptide fragment having an amino acid sequence sharing at least 80% sequence identity with one of SEQ ID Nos: 16-21 and a function of the polypeptide fragment in a). Specifically, the polypeptide fragment in b) refers to a polypeptide fragment obtained by substitution, deletion or addition of one or more (for example, 1-50, 1-30, 1-20, 1-10, 1-5, or 1-3) amino acids in an amino acid sequence selected from SEQ ID Nos: 16-21 and having the function of the polypeptide fragment that has the amino acid sequence selected from SEQ ID Nos: 16-21, or a polypeptide fragment obtained by adding one or more (for example, 1-50, 1-30, 1-20, 1-10, 1-5, or 1-3) amino acids at the N-terminal and/or C-terminal of an amino acid sequence selected from SEQ ID Nos: 16-21 and having the function of the polypeptide fragment that has the amino acid sequence selected from SEQ ID Nos: 16-21. This function can be an ability to bind specifically to PD-L1, to block PD-L1/PD-1 interaction, so as to block the PD-L1/PD1 pathway, to enhance IFN-γ and/or IL-2 expression in T lymphocytes, or to inhibit tumor growth. The anti-PD-L1 single domain antibody fragment in b) has an amino acid sequence sharing at least 80%, 85%, 90%, 93%, 95%, 97%, or 99% sequence identity with one of SEQ ID Nos: 16-21. The anti-PD-L1 single domain antibody fragment may typically be derived from *Vicugna pacos*, e.g., the CDR region thereof may be derived from *Vicugna paces.* The anti-PD-L1 single domain antibody fragment may typically be humanized, for example, the FR regions thereof may be derived from homo sapiens.

As used herein, sequence identity refers to two or more sequences that have a specified percentage of nucleotides or amino acid residues that are the same. The sequence identity of two or more sequences can be calculated by using computational software well known in the art, such software may be, for example, NCBI.

The fusion protein provided by the present disclosure may include the anti-VEGF fragment. The anti-VEGF fragment described above may typically be a peptide or protein fragment antagonizing VEGF. For example, the anti-VEGF fragment may be a monoclonal antibody or the like. For another example, the anti-VEGF fragment may be Bevacizumab.

In a specific embodiment of the present disclosure, the anti-VEGF fragment includes:

c) a polypeptide fragment having an amino acid sequence selected from SEQ ID Nos: 22-23; or

d) a polypeptide fragment having an amino acid sequence sharing at least 80% sequence identity with one of SEQ ID Nos: 22-23 and having a function of the polypeptide fragment in c). Specifically, the polypeptide fragment in d) refers to a polypeptide fragment obtained by substitution, deletion or addition of one or more (for example, 1-50, 1-30, 1-20, 1-10, 1-5, 1-3, 1, 2, or 3) amino acids in an amino acid sequence selected from SEQ ID Nos: 22-23 and having the function of the polypeptide fragment that has the amino acid sequence selected from SEQ ID Nos: 22-23, or a polypeptide fragment obtained by adding one or more (for example, 1-50, 1-30, 1-20, 1-10, 1-5, 1-3, 1, 2, or 3) amino acids at the N-terminal and/or C-terminal of an amino acid sequence selected from SEQ ID Nos: 22-23 and having the function of the polypeptide fragment that has the amino acid sequence selected from SEQ ID Nos: 22-23. This function can be an ability to antagonize VEGF, to prolong the half-life *in vivo* because an Fc fraction that binds to an FcRn receptor is included, or to kill cancer cells by binding to effector cells expressing Fc receptors. The polypeptide fragment in d) of the anti-VEGF fragment has an amino acid sequence sharing at least 80%, 85%, 90%, 93%, 95%, 97%, or 99% sequence identity with one of SEQ ID Nos: 22-23. The anti-VEGF fragment may typically be derived from *Mus musculus,* e.g., the CDR region thereof may be derived from *Mus musculus.* The anti-VEGF fragment may typically be humanized, for example, the FR region thereof may be derived from homo sapiens.

The fusion protein provided by the present disclosure may include the TGF-β binding fragment. The above-mentioned TGF-β binding fragment can specifically bind to various TGF-β isomers (e.g., TGF-β1, TGF-β2, and TGF-β3, etc.) that are often highly expressed in a variety of malignant tumors and that are one of the important factors leading to poor clinical outcomes. For example, the TGF-β binding fragment may be a TGF-βRII (TGF-β receptor II) extracellular region fragment.

In a specific embodiment of the present disclosure, the TGF-β binding fragment includes:
e) a polypeptide fragment having an amino acid sequence of SEQ ID No: 24; or
f) a polypeptide fragment having an amino acid sequence sharing at least 80% sequence identity with SEQ ID No: 24 and having a function of the polypeptide fragment in e). Specifically, the polypeptide fragment in f) refers to a polypeptide fragment obtained by substitution, deletion or addition of one or more (for example, 1-50, 1-30, 1-20, 1-10, 1-5, 1-3, 1, 2, or 3) amino acids in the amino acid sequence of SEQ ID No: 24 and having the function of the polypeptide fragment that has the amino acid sequence of SEQ ID No: 24, or a polypeptide fragment obtained by adding one or more (for example, 1-50, 1-30, 1-20, 1-10, 1-5, 1-3, 1, 2, or 3) amino acids at the N-terminal and/or C-terminal of the amino acid sequence of SEQ ID No: 24 and having the function of the polypeptide fragment that has the amino acid sequence of SEQ ID No: 24. This function can be an ability to bind to various TGF-β isomers (e.g., TGF-β1, TGF-β2 and TGF-β3, etc.), so as to remove over-expressed TGF-β from tumor microenvironment, or to enhance the killing function of tumor-infiltrating T cells against tumor cells. The polypeptide fragment in f) of the TGF-β binding fragment has an amino acid sequence that is at least 80%, 85%, 90%, 93%, 95%, 97%, or 99% identical to SEQ ID No: 24. The TGF-β binding fragment can typically be derived from homo sapiens.

The fusion protein provided by the present disclosure may include a linker peptide fragment. The fusion protein may usually include a plurality of linker peptide fragments, and the linker peptide fragments may be provided among at least a part of the domains or all the domains. For example, the linker peptide fragment is provided between the anti-PD-L1 single domain antibody fragment and the anti-VEGF fragment. For another example, the linker peptide fragment is provided between the anti-VEGF fragment and the TGF-β binding fragment. The linker peptide fragment may usually be a flexible peptide with suitable length and rich in G, S, and/or A (consisting primarily of glycine (G), serine (S), and/or alanine (A)), so that adjacent protein domains are free to move relative to each other. For example, an amino acid sequence of the linker peptide fragment may include (GS)ₙ, (GGS)ₙ, (GGSG)ₙ, (GGGS)ₙA, (GGGGS)ₙA, (GGGGS)ₙG, (GGGGA)ₙA, (GGGGG)ₙA, and the like, wherein n is an integer ranging from 1 to 10. In a specific embodiment of the present disclosure, the length of the amino acid sequence of the linker peptide fragment may be 3-30, 3-4, 4-6, 6-8, 8-10, 10-12, 12-14, 14-16, 16-18, 18-20, 20-22, 22-24, 24-26, 26-28, or 28-30. In another specific embodiment of the present disclosure, the linker peptide fragment may include a polypeptide fragment having an amino acid sequence selected from SEQ ID Nos: 34-36.

The fusion protein provided by the present disclosure may be linear, for example, the fusion protein may sequentially include the anti-PD-L1 single domain antibody fragment, the anti-VEGF fragment, and the TGF-β binding fragment from the N-terminus to the C-terminus. The fusion protein may also have a structure similar to that of a monoclonal antibody, for example, the anti-PD-L1 single domain antibody fragment may be located at the N-terminus of the heavy chain of the anti-VEGF fragment, for another example, the anti-PD-L1 single domain antibody fragment may be located at the N-terminus of the light chain of the anti-VEGF fragment, and for another example, the TGF-β binding fragment may be located at the C-terminus of the heavy chain of the anti-VEGF fragment. In a specific embodiment of the present disclosure, the fusion protein has an amino acid sequence including one of SEQ ID NO: 23 and SEQ ID NOs: 25-33, for example, the fusion protein may have an amino acid sequence including SEQ ID NO: 25 and SEQ ID NO: 26, the fusion protein may have an amino acid sequence including SEQ ID NO: 25 and SEQ ID NO: 27, the fusion protein may have an amino acid sequence including SEQ ID NO: 25 and SEQ ID NO: 28, the fusion protein may have an amino acid sequence including SEQ ID NO: 25 and SEQ ID NO: 29, the fusion protein may have an amino acid sequence including SEQ ID NO: 30 and SEQ ID NO: 27, the fusion protein may have an amino acid sequence including SEQ ID NO: 30 and SEQ ID NO: 29, the fusion protein may have an amino acid sequence including SEQ ID NO: 31 and SEQ ID NO: 23, the fusion protein may have an amino acid sequence including SEQ ID NO: 32 and SEQ ID NO: 23, or the fusion protein may have an amino acid sequence including SEQ ID NO: 33 and SEQ ID NO: 23.

A second aspect of the present disclosure further provides an isolated polynucleotide encoding the fusion protein provided in the first aspect of the present disclosure. The polynucleotide may be RNA, DNA, cDNA, or the like. Methods for preparing such isolated polynucleotide are known to those skilled in the art. For example, the isolated polynucleotide may be prepared by automatic DNA synthesis and/or recombinant DNA techniques, and may be isolated from a suitable natural source.

A third aspect of the present disclosure provides a construct, including the isolated polynucleotide provided in the second aspect of the present disclosure. Methods for preparing such construct are known to those skilled in the art. For example, the construct may be obtained by methods such as *in vitro* recombinant DNA technology, DNA synthesis technology, *in vivo* recombinant technology, and more specifically, may be obtained by inserting the isolated polynucleotide described above into the multiple cloning site of an expression vector. The expression vector in the present disclosure generally refers to various commercially available expression vectors well known in the art and the like, for example, the expression vector may be a bacterial plasmid, a phage, a yeast plasmid, a plant cell virus, an animal cell virus (such as adenovirus), a retrovirus, or other vectors. Generally, a suitable vector includes a replication origin, a promoter sequence, a convenient restriction enzyme cut site, and one or more selectable tags that function in at least one organism. For example, the promoters may include but are not limited to: lac or trp promoter of *E.coli*; PL promoter of λ phage; and eukaryotic promoter (including immediate early cytomegalovirus (CMV) promoter, herpes simplex virus (HSV) thymidine kinase promoter, simian virus 40 (SV40) early promoter, simian virus 40 (SV40) late promoter, methanol oxidase promoter of Pichia pastoris, and other known promoters that control gene expression in prokaryotic cells, eukaryotic cells, or viruses). Marker genes may provide phenotypic characters of host cells used for selection and transformation, for example, marker genes can include but are not limited to dihydrofolate reductase, neomycin resistance gene, and green fluorescent protein (GFP) used for eukaryotic cell culture, or tetracycline or ampicillin resistance gene used for *E*. *coli*, etc. When expressing the polynucleotide, the expression vector may further include an enhancer sequence, and an insertion of the enhancer sequence results in an enhanced transcription. Enhancer is a cis-acting element of DNA, which typically contains about 10 to 300 base pairs and acts on a promoter to enhance the transcription of genes.

A fourth aspect of the present disclosure provides an expression system, including the construct provided in the third aspect of the present disclosure or the isolated polynucleotide provided in the second aspect of the present disclosure which is incorporated into a genome for expression of the above fusion protein. The expression system may be a host cell, and any cell suitable for the expression of the expression vector can serve as a host cell. For example, the host cell may be a prokaryotic cell (such as a bacterial cell), a lower eukaryotic cell (such as a yeast cell), a filamentous fungal cell, or a higher eukaryotic cell (such as a mammalian cell). Representative examples include: *E*. *coli*, Streptomyces, bacterial cells of Salmonella typhimurium, fungal cells (such as yeast), filamentous fungi, plant cells, insect cells of Drosophila S2 or Sf9, animal cells of CHO, COS, 293 cells or Bowes melanoma cells, and the like. Methods for introducing constructs into host cells are known to those skilled in the art, for example, microinjection, biolistics, electroporation, virus-mediated transformation, electron bombardment, calcium phosphate precipitation, and the like.

A fifth aspect of the present disclosure provides a method for preparing the fusion protein provided in the first aspect of the present disclosure, and those skilled in the art can select a suitable method for preparing the fusion protein. For example, the method for preparing the fusion protein includes culturing the expression system provided in the fourth aspect of the present disclosure under a condition suitable for expressing the fusion protein, collecting all culture materials containing the fusion protein, and performing isolation and purification to provide the fusion protein.

A sixth aspect of the present disclosure provides a use of the fusion protein provided in the first aspect of the present disclosure or a culture of the expression system provided in the fourth aspect of the present disclosure in the preparation of medications. The medication may be used for treating tumors. The tumor may be, for example, cancer or solid tumor, specifically may be lung cancer, melanoma, gastric cancer, ovarian cancer, colon cancer, liver cancer, renal carcinoma, bladder cancer, breast cancer, classical Hodgkin's lymphoma, hematological malignancy, sarcoma, head and neck cancer, nasopharyngeal carcinoma, etc. These cancers may be in the early, middle, or late stage, such as metastatic cancer.

A seventh aspect of the present disclosure provides a pharmaceutical composition including the fusion protein provided in the first aspect of the present disclosure or a culture of the expression system provided in the fourth aspect of the present disclosure. The pharmaceutical composition includes a therapeutically effective amount of the fusion protein or the culture of the expression system. In the present disclosure, "therapeutically effective amount" generally refers to a dosage that, after an appropriate period of administration, results in an alleviation of the severity of disease symptoms, an increase in the frequency and duration of asymptomatic periods of disease, or the prevention of impairment or incapacity due to disease suffering. The ability to inhibit tumor growth can be evaluated in an animal model system that is predictive of efficacy against human tumors. Alternatively, the ability to inhibit tumor growth can be assessed by examining the ability to inhibit cell growth, which can be determined *in vitro* by assays well known to those skilled in the art. A therapeutically effective amount of the fusion protein or the pharmaceutical composition is generally capable of reducing the size of a tumor, or otherwise ameliorating a symptom of a subject. An appropriate "therapeutically effective amount" in any individual case can be determined by one of ordinary skills in the art. For example, the exact amount required depends on the age of the subject, the severity of the condition being treated, the selected composition, and the administration route. Prescription (for example, dosage and the like) for treatment can be determined by a physician, and typically factors that need to be taken into account include but are not limited to the to-be-treated disease, the condition of the patient, the to-be-delivered site, the administration route, and other factors.

A pharmaceutically acceptable carrier may also be included in the pharmaceutical composition provided by the present disclosure. The carrier may include a variety of excipients and diluents, which are not essential active ingredients and do not have unduly toxic upon administration. Suitable carriers should be well known to those skilled in the art, for example, a full discussion of the pharmaceutically acceptable carrier can be found in Remington's Pharmaceutical Sciences (Mack Pub. Co., N.J., 1991).

An eighth aspect of the present disclosure provides a treating method, including administering a therapeutically effective amount of the fusion protein provided in the first aspect of the present disclosure, a culture of the expression system provided in the fourth aspect of the present disclosure, or the pharmaceutical composition provided in the seventh aspect of the present disclosure.

The term "treat" as used herein refers to an action that pharmaceutically and/or physiologically realizes prevention, cure, or amelioration of the disease. A good treatment effect means medically reducing one or more symptoms of the disease, completely eliminating the disease, arresting or delaying the onset of the disease, and/or mitigating the risk of disease development or disease progression.

The term "subject" as used herein typically includes human, non-human primate, and other mammals (e.g., dog, cat, horse, sheep, pig, cow, etc.) that may benefit from treatment with the formulation, kit, or combined preparation.

In the present disclosure, the fusion protein, culture material of the expression system, or pharmaceutical composition may be used as an active ingredient individually, or may be used in combination with other agents, so as to be administered in a combination therapy. For example, the multi-domain fusion protein, the culture of the expression system, or the pharmaceutical composition having anticancer activities may be administered in combination with at least one other antitumor drug. For another example, the multi-domain fusion protein, the culture of the expression system, or the pharmaceutical composition having anticancer activities may be administered in combination with an antibody targeting other tumor-specific antigens.

The multi-domain fusion protein with an anticancer activity provided by the present disclosure combines the functions of the anti-PDL1 monoclonal antibody, the anti-VEGF monoclonal antibody, and the TGF-β receptor, where the anti-PDL1 monoclonal antibody blocks the PD-L1/PD-1 interaction, the anti-VEGF monoclonal antibody inhibits microvascular growth and tumor metastasis, and the TGF-β receptor relieves cancer cells from TGF-β signal tolerance and enhances immune response. The fusion protein molecule has an excellent tumor suppression effect and a good prospect of industrialization.

The embodiments of the present disclosure are illustrated by specific examples below, and those skilled in the art can easily understand other advantages and effects of the present disclosure from the contents disclosed in the specification. The present disclosure can also be implemented or applied through other different specific embodiments. Various modifications or changes can also be made to all details in the specification based on different points of view and applications without departing from the spirit of the present disclosure.

Before the detailed description of the embodiments of the present disclosure, it needs to understand that the protection scope of the present disclosure is not limited to the specific exemplary embodiments described below. It should further understand that the specific terms used in the embodiments are just for the description of the present disclosure, rather than limiting the protection scope of the present disclosure.

When numerical ranges are given in the description, it should understand that, unless otherwise indicated herein, both endpoints of each numerical range and any number between the two endpoints may be selected for use. Unless otherwise defined, all technical and scientific terms used in this disclosure have the same meaning as understood by those skilled in the art from the prior art. In addition, except for the methods, devices, and materials used in the embodiments, the present disclosure can also be implemented by any methods, devices, and materials of the prior art similar or equivalent to those described in the embodiments.

Unless otherwise stated, the laboratory procedures, test methods, and production methods disclosed in the present disclosure are those well-known and commonly used in the field of molecular biology, biochemistry, chromatin biology, analytical chemistry, cell culture, recombinant DNA, and other routine techniques. These methods have been well described in the existing literature, and please refer to Sambrook et al., MOLECULAR CLONING: A LABORATORY MANUAL, Second edition, Cold Spring Harbor Laboratory Press, 1989 and Third edition, 2001; Ausubel, et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol.304, Chromatin (P.M.Wassarman and A.P.Wolffe, eds.), Academic Press, San Diego, 1999; and METHODS IN MOLECULAR BIOLOGY, Vol.119, Chromatin Protocols (P.B.Becker, ed.) Humana Press, Totowa, 1999, etc. for details.

### Embodiment 1 Construction and recombinant expression of fusion protein

According to the codon preference of Chinese hamster ovary (CHO) cells, the amino acid sequences of the multi-domain fusion protein, PD-L1-Fc fusion protein (SEQ ID NO: 47), and Fc- TGF β RII fusion protein (SEQ ID NO: 48) in Table 1 were converted into base sequences, respectively. *Hin*dIII restriction enzyme cutting site and Kozak sequence (GCCACC) were introduced to 5' end of the coding sequences of heavy and light chains, and termination codon and EcoRI restriction enzyme cutting site were introduced to 3' end of the coding sequences of heavy and light chains, so as to obtain a full-length DNA by gene synthesis (General Biol (Anhui) Co., ltd.). The synthesized coding genes of heavy chains and light chains were subjected to Hindlll-HF (purchased from NEB, R3104V) and EcoRI-HF (purchased from NEB, R3101V) double digestion, respectively, and the agarose gel DNA/PCR product miniprep purification and recovery kit (purchased from Biomiga) was used for gel cutting and recovery. The recovered DNA was ligated with the pCDNA3.1(+) vector through T4 ligase (purchased from NEB, M0202V), where the pCDNA3.1(+) vector was also subjected to Hindlll and EcoRI double digestion. The recovered DNA ligated with the pCDNA3.1(+) vector was transformed to Top10 competence, and then spread on an LB ampicillin-resistant plate for culturing. Clones were picked for sequencing, and heavy chain and light chain expression plasmids based on pCDNA3.1(+) were constructed, respectively. Heavy chain and light chain expression plasmids were extracted separately with EndoFree Plasmid Maxi Kit (purchased from Biomiga, BW-PD3511-02), respectively, and then mixed at a ratio of 1:1. 1.0mg of a mixture of the plasmids was diluted with 25ml of Wayne293 expression medium (purchased from QuaCell Biotechnology, Co., Ltd, A21501). 3.0mg of PEI (linear, 25KD, Polysciences, Inc.) was also diluted with 25mL of Wayne293 expression medium, then added to the mixture of the plasmids for mixing well, and afterward, incubated for 30 min at room temperature. Hek293F cells (cell viability is greater than 95%) in the logarithmic phase were collected and counted, then centrifuged for 10 min at 1100 rpm. The supernatant was discarded and Hek293F cells were resuspended in 450mL of Wayne293 expression medium. The above plasmid -PEI mixture was added to the cell suspension, then cultured in a 5% CO₂ incubator shaker at 37°C for 7 days, and afterward centrifuged to obtain the supernatant for subsequent protein purification.

**Table 1 Amino acid sequence and coding sequence of multi-domain fusion protein**

| | Name | Amino acid sequence (heavy chain/light chain SEQ ID NO:) | DNA sequence (heavy chain/light chain SEQ ID NO:) |
|---|---|---|---|
| N-terminus of light chain | TAF-5 | 25, 26 | 37, 38 |
| | TAF-5a | 25, 27 | 37, 39 |
| | TAF-5b | 25, 28 | 37, 40 |
| | TAF-5c | 25, 29 | 37, 41 |
| | TAF-7 | 30, 27 | 42, 39 |
| | TAF-8 | 30, 29 | 42, 41 |
| N-terminus of heavy chain | TAF-6 | 31, 23 | 43, 46 |
| | TAF-6a | 32, 23 | 44, 46 |
| | TAF-6b | 33, 23 | 45, 46 |

### Embodiment 2 Purification of multi-domain fusion protein

### 2.1 The anti-PD-L1 single domain antibody is located at the N-terminus of the heavy chain of the anti-VEGF monoclonal antibody

After the pH of the cell fermentation supernatant was adjusted to 7.0, the sample was loaded to the Protein A affinity chromatography column (Bestchrom Biosciences Co., Ltd.) and eluted with 100% 0.1M Gly-HCl (pH = 3.0), where the equilibrium liquid was 20mM PB, 0.15M NaCl (pH = 7.0), and the eluant was previously added with 10% 1M Tris-HCl (pH = 8.5). The 100% 0.1M Gly-HCl (pH = 3.0) eluant was then diluted until the conductivity is less than 3 ms/cm. The pH of the cell fermentation supernatant obtained after elution was adjusted to 7.0, and the sample was loaded onto a DSP chromatographic column (Bestchrom Biosciences Co., Ltd.) for another elution. 15% and 100% (20 mM PB, 0.5 M NaCl, pH 7.0) served as eluants, respectively, and the component obtained after elution with the 15% eluant is the target protein. The protein concentration was determined by UV280 method.

### 2.2 The anti-PD-L1 single domain antibody is located at the N-terminus of the light chain of the anti-VEGF monoclonal antibody

After the pH of the cell fermentation supernatant was adjusted to 7.0, the sample was loaded to the Protein A affinity chromatography column (Bestchrom Biosciences Co., Ltd.) and eluted with 100% 0.1M Gly-HCl (pH = 3.0), where the equilibrium liquid was 20mM PB, 0.15M NaCl (pH = 7.0), and the eluant was previously added with 10% 1M Tris-HCl (pH = 8.5). The 100% 0.1M Gly-HCl (pH = 3.0) eluant was then diluted until the conductivity is 4 ms/cm. The cell fermentation supernatant obtained after elution was loaded on a Super Q (TOSOH) chromatography column and then diluted with 35 % 500mM NaCl 20mM Tris (pH = 8.0) and 100% 500mM NaCl 20mM Tris (pH = 8.0), respectively, where the equilibrium liquid is 20mM Tris pH8, and the component obtained after elution with the 35% eluant is the target protein after removing excess light chain by flowthrough mode. The protein concentration was determined by UV280 method.

The protein purity was tested by SEC-HPLC-UV analysis, where the detector is Agilent 1100 LC, the detection wavelength is 214 nm, the mobile phase is 150 mM pH 7.0 PB + 5% isopropanol; the column is Superdex 200 Increase 5/150 GL, the run time is 15 minutes, and the column temperature is 25°C. The test results show that the protein purity is greater than 95%.

Purification of PD-L1-Fc fusion protein (SEQ ID NO: 47) and Fc-TGF β RII fusion protein (SEQ ID NO: 48): after the pH of the cell fermentation supernatant was adjusted to 7.0, the sample was loaded to the Protein A affinity chromatography column (Bestchrom Biosciences Co., Ltd.) and eluted with 100% 0.1M Gly-HCl (pH = 3.0), where the equilibrium liquid was 20mM PB, 0.15M NaCl (pH = 7.0), and the eluant was previously added with 10% 1M Tris-HCl (pH = 8.5) for neutralization.

### Embodiment 3 Identification of in vitro viability of multi-domain fusion protein

### 3.1 In vitro viability assay of anti-PD-L1 single domain antibody fragment

The CD5L-OKT3scFv-CD14 gene sequence (GenBank: ADN42857.1) was synthesized, digested with *Hin*dIII-*Eco*RI (Takara), and inserted into the vector pCDNA3.1 to construct pCDNA3.1-antiCD3TM. The human PD-L1 gene (GenBank: NM_014143.2) was used as a template, and the PD-L1 fragment obtained by high-fidelity amplification was recombinantly connected and inserted into pCDNA3.1-antiCD3TM to construct pCDNA3.1-antiCD3TM-PDL1. CHO cells (Thermo) were transfected with pCDNA3.1-antiCD3TM-PDL1, and G418 selection was performed for 10-14 days to generate a stable CHO-antiCD3TM-PDL1 cell line.

The human PD-L1 gene (GenBank: NP_005009.2) was used as a template for amplification, and the resulting fragment was recombinantly connected to PB513B1-dual-puro vector (YouBio) to construct the plasmid pB-PD1, where the PB513B1-dual-puro vector was previously subjected to *Hin*dIII-BamHI (Takara) digestion. pGL4.30 (YouBio) was used as a template, and the fragment obtained by high-fidelity amplification was recombinantly connected to pB-PD1 vector to construct pB-NFAT-Luc2p-PD1 plasmid, where the pB-PD1 vector was previously subjected to Sfil-Xba (I Takara) digestion. After the successful construction of the plasmids, EndoFree Plasmid Maxi Kit (Biomiga) was employed to extract these plasmids for transfection of Jurkat cells (Stem Cell Bank of Chinese Academy of Sciences). Referring to the method in patent CN 107022571A, Jurkat cells were treated into a relatively adherent status by using 0.1 mg/ml poly-D-lysine and then transfected according to the transfection instructions in liposomal transfection kit (Lipofectamine 3000; invitrogen). On the third day, RPMI1640 medium (Thermo) containing 10% FBS and 2.5µg/ml of puromycin was used for pressurized screening. Afterward, the medium was supplemented at regular intervals. The content of puromycin was gradually increased to 4µg/ml after the cell viability was restored, and finally, a monoclonal Jurkat-NFAT-Luc2p-PD1 cell line was obtained.

CHO-antiCD3TM-PDL1 and Jurkat-NFAT-Luc2p-PD1 cells were collected and counted, and the cell density was adjusted to 4×10⁶/ml. 25µl of each kind of cell was added into each well of the 96-well plate. The fusion protein samples prepared in Embodiment 2 were gradiently diluted with 1% BSA, respectively, and 50µl of these diluted fusion protein samples were added to the cells. After 6 hours of incubation at 37°C and 5% CO₂, 10µL of luciferase substrate (Promega, E2620) was added to each well. The cells were oscillated on a shaker for 2 min before counting. Operations may be performed as described in the kit.

### 3.2 In vitro viability assay of anti-VEGF fragment

HEK293 cells were spread on a 6-well cell culture plate and incubated overnight in a 5% CO₂ incubator at 37°C, where 1.0×10⁶ cells were added per well. According to the instruction of Lipofectamine^{®} 3000 Transfection Reagent, 1.0µg of pcDNA-KDR plasmid and 4µg of pGL4.30 plasmid served as a transfection system. After 48 hours of transfection, the cells were transferred to a 10cm cell culture dish, and 200µg/ml of G418 and 100µg/ml of Hygromycin were added. The cell culture medium was replaced every 3 days until clone groups were formed. After cell detachment, the cells were spread on a 96-well cell culture plate, and once monoclonal cells were formed, 0.1µg/ml of VEGF was used for stimulation for 6 h. The chemiluminescence was detected, the monoclonal cells with obvious signal response were selected for further culturing, and finally, a monoclonal cell line HEK293-NFAT-KDR was obtained. HEK293-NFAT-KDR cells were plated at a cell density of 4 million/well, detached with Accutase, and collected and centrifuged at 1000 rpm for 5 min. The supernatant was discarded, and an analysis culture solution (DMEM + 5% FBS) was added for resuspension. The cells were counted and the cell density was adjusted to 1.6×10⁶/ml. 25ul of the cells with a density of 1.6×10⁶/ml was added to each well of a 96-well cell culture plate. VEGF solution with a concentration of 60ng/ml was prepared by using the analysis culture solution and 25ul of the VEGF solution was added to each well. Fusion proteins in Embodiment 2 were prepared by using the analysis culture solution and 25ul of the fusion protein was added to each well of the cell culture plate. The cell culture plate was incubated for 6h under a condition of 5% CO₂ and 37°C. 10ul of Bright-Glo luciferase detection reagent (Promega, E2620) was added to each well, and the cell culture plate was oscillated for 2 min. 80ul of lysis buffer was transferred to the white assay plate for counting by using the microplate reader.

### 3.3 In vitro viability assay of TGF- β binding fragment

The mouse breast cancer cells 4T1 were cultured until reaching approximately 90% confluence (in a 10 cm dish), then dissociated using trypsin. The cells were plated with a density of 4×10⁵ cells per well in a 6-well plate and incubated overnight. 4T1 cells were transfected with the extracted pGL4.48 [luc2P SBE Hygro] plasmid sample by using Lipofectamine^{®} 3000. After 24h of transfection, the obtained TGFβ-4T1 cells were digested with trypsin and transferred to a 10cm-culture dish. The cells were then subjected to pressure selection using RPMI 1640 culture medium containing 10% FBS and 150ug/ml of hygromycin (InvivoGen, Cat no.: ant-hg-1). After 10-15 days of pressure selection, TGFβ-4T1 cells were plated at a cell density of 2 cells/well for subsequent monoclonal screening. TGFβ1 (Novoprotein, 10ug, Cat no.: CA59) was utilized to stimulate the monoclonal cells to verify the transfection effect of the monoclonal cells, and finally, the TGFβ-4T1 monoclonal cells were obtained.

The TGFβ-4T1 cells were cultured until reaching approximately 90% confluence. Approximately 2.5 ml of 0.25% trypsin was added to digest the cells, and the digestion was carried out at room temperature for 2 minutes. The cells attached to the culture dish were detached and dispersed in the trypsin solution. 5 min later, a complete culture medium (RPMI 1640 + 10% FBS) was added to terminate the digestion and the cells were blown continuously until an even distribution is achieved. The cells were transferred to a 50 ml centrifuge tube and centrifuged at 1000 rpm for 5 min. The supernatant was discarded, 2 ml of the complete culture medium was added to resuspend the cells, and the cell density was measured by using a cytometer. The cells were diluted with the complete culture medium (RPMI 1640 +10% FBS), so that the cell density was 2×10⁵/ml. 100ul of the diluted cells with a density of 2×10⁵ /ml was added to each well of a 96-well plate, and the cell density in each well was 2×10⁴/well. The 96-well plate was cultured in an incubator at 37°C overnight. The fusion protein samples prepared in Embodiment 2 were diluted to a specified concentration (after dilution, each protein sample is placed at room temperature for 1h) by using RPMI 1640 + 0.2% FBS medium (containing 2ng/ml TGFβ1). After being cultured overnight in an incubator at 37°C, the supernatant of the TGFβ-4T1 cells was discarded, 50ul of RPMI 1640 +0.2% FBS culture medium was added, and then 50µl of protein solutions with different concentrations were added. After the 96-well plate was placed in an incubator at 37°C for 3h, 10ul of Bright-Glo luciferase detection reagent (Promega, E2620) was added. The 96-well plate was oscillated for 3 min for counting by using the microplate reader.

The measurement results of the anti-PD-L1 activity, anti-VEGF activity, and anti-TGFβ activity of each multi-domain fusion protein were shown in Table 2. As can be seen from Table 2, each multi-domain fusion protein exhibited significant *in vitro* cell activity.

**Table 2 In vitro cell viability of multi-domain fusion protein**

| | Name | Anti-PD-L1 activity (IC50,nM) | Anti-VEGF activity (IC50,nM) | Anti-TGFβ activity (IC50,nM) |
|---|---|---|---|---|
| N-terminus of light chain | TAF-5 | 1.823 | 0.534 | 0.205 |
| | TAF-5a | 1.708 | 0.547 | 0.261 |
| | TAF-5b | 1.934 | 0.489 | 0.192 |
| | TAF-5c | 1.777 | 0.589 | 0.245 |
| | TAF-7 | 1.724 | 0.465 | 0.227 |
| | TAF-8 | 2.243 | 0.511 | 0.199 |
| N-terminus of heavy chain | TAF-6 | 1.674 | 0.509 | 0.201 |
| | TAF-6a | 1.814 | 0.444 | 0.234 |
| | TAF-6b | 1.716 | 0.540 | 0.213 |

### Embodiment 4 Pharmacokinetics of multi-domain fusion protein in C57BL/6 mice

C57BL/6 mice were divided into 4 groups, which were Avastin (Roche) low-dose (1 mg/kg) and high-dose (10 mg/kg) groups and TAF-6 low-dose (1 mg/kg) and high-dose (10 mg/kg) groups, respectively. Each group contained 8 mice, half of which are male, and another half of which are female. A single dose of drug was administered to mice by tail vein injection, and pharmacokinetic samples were collected at various time points in a cross-over manner. The samples were collected before administration, 1h, 6h, 24h, 48h, 78h, 120h, 144h, 168h, 192h, and 216h after administration, respectively. Serum drug concentrations in the pharmacokinetic samples were quantitatively determined using the ELISA method. VEGF was coated on the plate, HRP-labeled Goat anti-Human IgG Fc served as the secondary antibody, and TMB method was employed for detection, specifically, according to the relationship between signal and concentration of standard curve, concentration was obtained by regression, and the main pharmacokinetic parameters were calculated by a non-compartment model of PK Solver software.

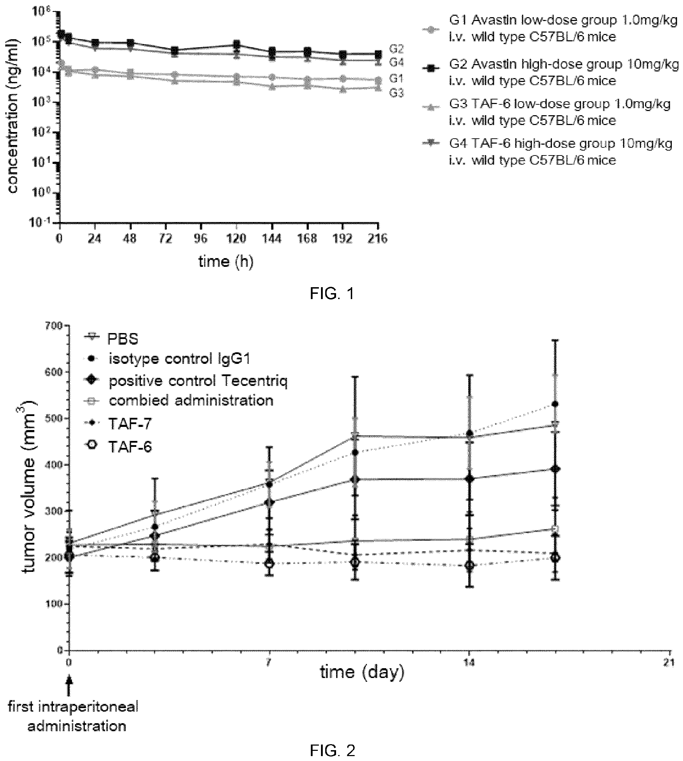

The results, as shown in FIG. 1, indicated that the pharmacokinetic parameters were similar for C57BL/6 mice treated with Avastin and TAF-6 at high and low doses groups. The ratio of administration dosage in the Avastin low-dose group to Avastin high-dose group was 1:10, the ratio of Cₘₐₓ in the Avastin low-dose group to Avastin high-dose group was 1:9.3, the ratio of AUCₗₐₛₜ in the Avastin low-dose group to Avastin high-dose group was 1:8.9, and Tₘₐₓ of the Avastin low-dose group and Avastin high-dose group were both 1 h. The exposure dose (Cₘₐₓ and AUCₗₐₛₜ) increased proportionally with the dose administered. The ratio of administration dosage in the TAF-6 low-dose group to TAF-6 high-dose group was 1:10, the ratio of Cₘₐₓ in the TAF-6 low-dose group to TAF-6 high-dose group was 1:8.6, the ratio of AUCₗₐₛₜ in the TAF-6 low-dose group to TAF-6 high-dose group was 1:8.5, and Tₘₐₓ of the TAF-6 low-dose group and TAF-6 high-dose group were both 1 h. The increase in exposure levels (Cₘₐₓ and AUCₗₐₛₜ) showed a dose-proportional increase. Therefore, AUC and Cₘₐₓ were linearly correlated with the dose administered between Avastin high-dose and low-dose groups and TAF-6 high-dose and low-dose groups. The difference in AUC between Avastin and TAF-6 may be attributed to the target-mediated drug disposition (TMDD) effect mediated by the PD-L1 target.

### Embodiment 5 Antitumor activities of multi-domain fusion proteins in humanized mice transplanted with breast cancer MDA-MB-231 cells and PBMC.

MDA-MB-231 (human breast cancer) cells were used for modeling in immune system-humanized mice (M-NSG mouse) engrafted with human PBMC, so as to determine the *in vivo* efficacy of the multi-domain fusion proteins of the present disclosure. 6 to 8 weeks-old female M-NSG mice were inoculated with MDA-MB-231 cells (10*10E6 + matrigel 25%). PBMC (5*10E6/0.2ml) was injected into the mice through the tail vein on day 7, then the tumor volume and body weight were observed. Mice with a tumor volume between 140-260 mm³ were selected and randomly divided into 6 groups based on their tumor volumes and body weights. Each group contained 7 mice, and the mice were administered on the day of grouping. Tumor-bearing mice with excessively large or small tumor volumes were eliminated. PBS, isotype control IgG1, positive control Tecentriq (Roche), TAF-6, TAF-7, and combined administration (see Table 3 for details) were each injected intraperitoneally twice weekly for 3 weeks. PD-L1-Fc fusion protein (SEQ ID NO: 47) was a fusion protein of anti-PD-L1 single domain antibody and human IgG Fc (the anti-PD-L1 single domain antibody was located at the N-terminus of Fc), Fc-TGF β RII fusion protein (SEQ ID NO: 48) was a fusion protein of human IgG Fc and TGFβRII (TGFβRII was located at the C-terminus of Fc).

Prior to grouping and at the end of the experiment, blood samples were collected from the orbital vein of mice. FACS analysis results showed the presence of human CD45-positive cells in the peripheral blood of mice in each group, and the proportion of CD45-positive cells increased over time, indicating successful humanization of the mouse immune systems. The body weights (measured twice a week) of mice in group 3 and group 4 were almost stable during the experiment, and there was no mortality in the test animals. The tumor volumes (measured twice a week) of mice in group 3 and group 4 were smaller than those of mice in group 6 which adopted combined administration. The tumor volumes (measured twice a week) of mice in group 3 and group 4 were significantly smaller than those of mice in group 2 which only adopted Tecentriq, and the results were shown in FIG. 2.

**Table 3**

| Group | Sample to be administered | Dosage |
|---|---|---|
| Group 1 | Isotype control IgG1 | 10mg/kg |
| Group 2 | Positive control Tecentriq | 10mg/kg |
| Group 3 | **TAF-6** | 10mg/kq |
| Group 4 | **TAF-7** | 10mg/kg |
| Group 5 | PBS | 5ul/kg |
| Group 6 | Combined administration: PD-L1 -Fc fusion protein (SEQ ID NO:47), Bevacizumab (i.e., Avastin), Fc-TGFβRII fusion protein (SEQ ID NO:48) | Each is 10mg/kg |

### Embodiment 6 Antitumor activities of multi-domain fusion proteins in humanized mice transplanted with breast cancer MDA-MB-231 cells and PBMC.

Human-derived breast cancer MDA-MB-231 tumor was inoculated subcutaneously to the right anterior rib of female NCG mice, and PBMC cells were inoculated into mice a day later. When the tumor size reached about 53 mm³, mice were divided into 6 groups with 10 mice in each group. The groups were as follows: Vehicle group, TAF-6 low-dose (2 mg/kg, i.p., tiw × 9) group, TAF-6 medium-dose (6 mg/kg, i.p., tiw × 9) group, TAF-6 high-dose (18 mg/kg, i.p., tiw × 9) group, Tecentriq (4 mg/kg, i.p., tiw × 9) group, and Avastin (4 mg/kg, i.p., tiw × 9) group, and administration was performed on each group. Tumor volume and body weight were measured weekly, and the relationship between changes in body weight and tumor volume of tumor-bearing mice was recorded in relation to the duration of drug administration.

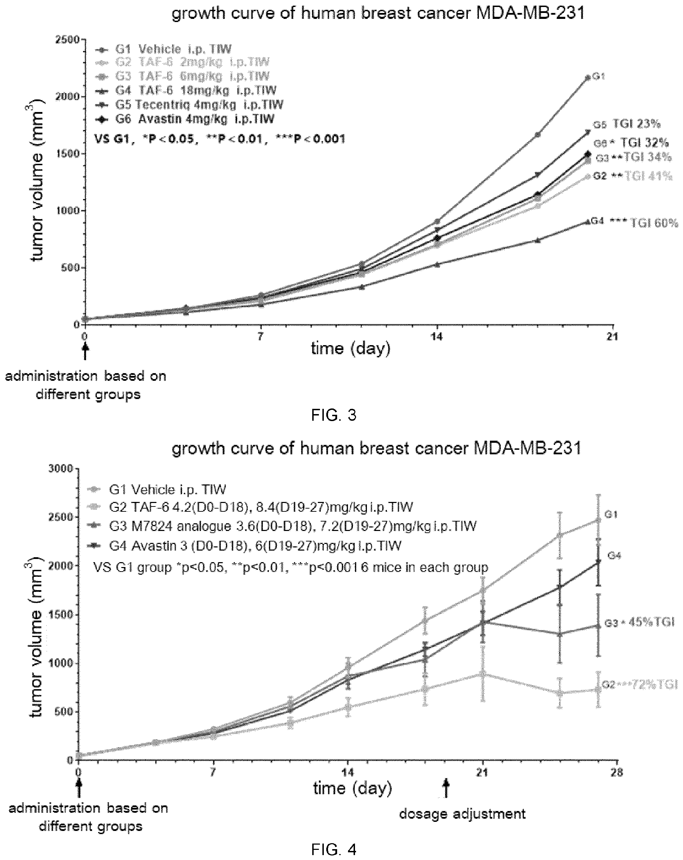

Blood was collected from the orbital vein of mice 2 days before the grouping and at the end of the experiment. FACS analysis results showed the presence of human CD45-positive cells in the peripheral blood of mice in each group, and the proportion of CD45-positive cells increased over time. At the end of the experiment, the tumor-bearing mice were euthanized, the tumors were weighed and photographed, and serum collection and tumor fixation were performed. The tumor growth inhibiting rate TGI_{TV} (%) was calculated and statistically analyzed, where the tumor growth inhibiting rates of TAF-6 low-dose group, TAF-6 middle-dose group, TAF-6 high-dose group, Tecentriq group, and Avastin group were 41%, 34%, 60%, 23%, and 32%, respectively. Except for Tecentriq group, the tumor volume in Vehicle group was significantly bigger than those in TAF-6 low-dose group, TAF-6 middle-dose group, TAF-6 high-dose group, and Avastin group (all p values were less than 0.05). The tumor volume in TAF-6 high-dose group was significantly smaller than that in the Tecentriq group (p<0.01), and there was no significant difference in tumor volume between the Tecentriq and Avastin groups (p>0.05). The results are shown in FIG. 3.

In summary, the drug TAF-6 had a significant anti-tumor effect on the humanized mouse model subcutaneously transplanted with MDA-MB-231 breast cancer cells and PBMC, which effectively inhibited tumor growth. The anti-tumor effect of TAF-6 was significantly better than that of Tecentriq, and the tumor inhibiting effect of TAF-6 was enhanced with the increase of dose.

In a separate experiment using the same tumor model, the tumor inhibiting activity of TAF-6 was compared with that of the positive control M7824 analog (prepared according to M7824 patent US9676863B2). Mice were divided into 4 groups with 6 mice in each group. The groups were as follows: Vehicle group, TAF6 (4.2 mg/kg, i.p., tiw × 8 times; later the dose was adjusted to 8.4 mg/kg, i.p., tiw × 4 times) group, M7824 analog (3.6 mg/kg, i.p., tiw × 8 times; later the dose was adjusted to 7.2 mg/kg, i.p., tiw × 4 times) group, and Avastin (3 mg/kg, i.p., tiw × 8 times; later the dose was adjusted to 6 mg/kg, i.p., tiw × 4 times) group. As shown in Fig. 4, the antitumor effect of TAF6 was significantly better than that of the M7824 analog at equimolar concentration doses.

### Embodiment 7 Antitumor activities of multi-domain fusion proteins in humanized mice transplanted with lung cancer Calu6 cells and PBMC

PBMC cells were inoculated into mice, and human-derived lung cancer Calu-6 cells were inoculated subcutaneously to the right anterior rib of male NCG mice four days later. When the tumor size reached about 50 mm³, mice were divided into 7 groups with 8 mice in each group. The groups were as follows: Vehicle group, TAF-6 low-dose (2 mg/kg, i.p., tiw × 10) group, TAF-6 medium-dose (7 mg/kg, i.p., tiw × 10) group, TAF-6 high-dose (25 mg/kg, i.p., tiw × 10) group, Tecentriq (5 mg/kg, i.p., tiw × 10) group, Avastin (5 mg/kg, i.p., tiw × 10) group, and Tecentriq+Avastin (5+5 mg/kg, i.p., tiw × 10) group, and administration was performed on each group. Tumor volume and body weight were measured weekly, and the relationship between changes in body weight and tumor volume in tumor-bearing mice was recorded in relation to the duration of drug administration. At the end of the experiment, the tumor-bearing mice were euthanized, the tumors were weighed and photographed, and serum collection and tumor fixation were performed. The tumor growth inhibiting rate TGI_{TV} (%) was calculated and statistically analyzed.

3 days before grouping and at the end of the experiment (PG-D23), blood was collected from the orbital vein of mice. FACS analysis results showed the presence of human CD45-positive cells in the peripheral blood of mice in each group, and the proportion of CD45-positive cells increased over time, indicating successful humanization of the mouse immune systems.

During the treatment period, the mice in each group were fed and watered normally, their body weights were generally stable, and there was no death of experimental animals.

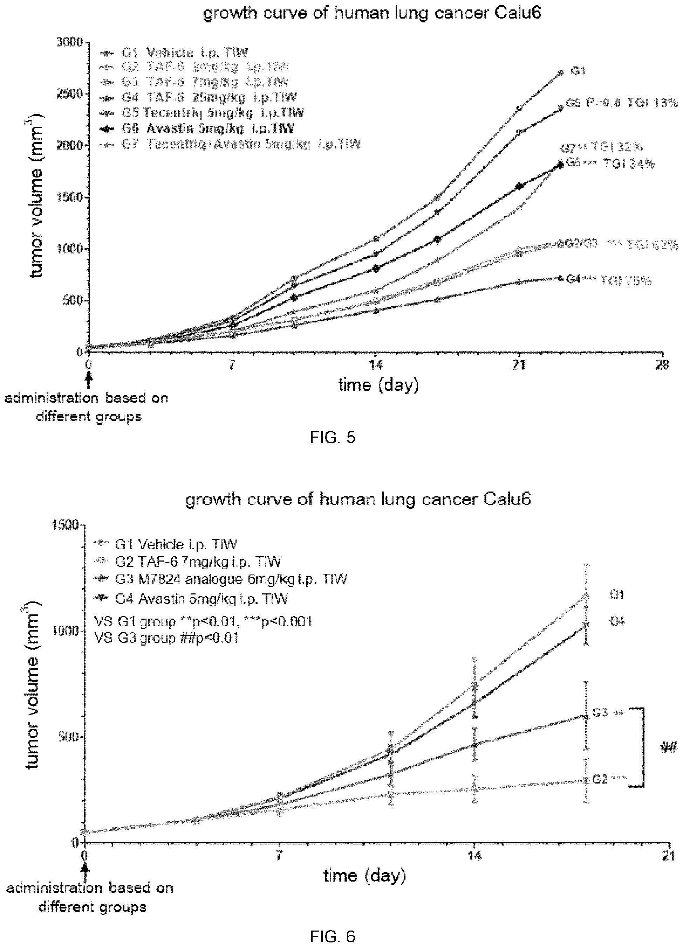

At the end of the experiment (PG-D23), the tumor growth inhibiting rates of TAF-6 low-dose group, TAF-6 medium-dose group, TAF-6 high-dose group, Tecentriq group, Avastin group, Tecentriq+Avastin group were 62%, 62%, 75%, 13%, 34%, and 32%, respectively. Except for Tecentriq group, the tumor volumes of TAF-6 low-dose group, TAF-6 medium-dose group, TAF-6 high-dose group, Avastin group, and Tecentriq+Avastin group were significantly smaller than that of the Vehicle group (all p were less than 0.01). The tumor volumes in TAF-6 low-dose, medium-dose, and high-dose groups were significantly smaller than those in Tecentriq, Avastin, and Tecentriq+Avastin groups, respectively (all p values were less than 0.01). Results were shown in the Table 4 and FIG. 5.

In summary, TAF-6 had a significant anti-tumor effect on the PBMC engrafted humanized mice model subcutaneously transplanted with lung cancer Calu-6 cells, which effectively inhibited tumor growth. The anti-tumor effect of TAF-6 was significantly better than that of Tecentriq, Avastin, Tecentriq+Avastin, and the tumor inhibiting effect of TAF-6 was enhanced with the increase in dose.

**Table 4 Antitumor effects of the test drugs on human lung cancer Calu6 (tumor volume)**

| **Group** | **The number of mice** | **Body weight (g) ^{a}** | | **Tumor volume (mm³)^{a}** | | **Tumor growth inhibiting rate TGI_{TV} (%)** |
|---|---|---|---|---|---|---|
| | | **Pre- administration (PG-DO)** | **After administration (PG-D23)** | **Pre- administration (PG-DO)** | **After administration (PG-D23)** | |
| Vehicle | 8 | 26.1±0.6 | 27.8±0.8 | 49±2 | 2707±95 | -- |
| TAF-6 low-dose | 8 | 26.1±0.4 | 26.3±0.7 | 49±3 | 1067±77 | 62% |
| TAF-6 medium-dose | 8 | 26.3±0.4 | 26.6±1.1 | 50±3 | 1050±61 | 62% |
| TAF-6 high-dose | 8 | 26.2±0.4 | 24.9±0.9 | 49±3 | 725±72 | 75% |
| Tecentriq | 8 | 26.3±0.5 | 24.4±0.8 | 50±3 | 2354±145 | 13% |
| Avastin | 8 | 26.2±0.6 | 27.3±0.8 | 50±3 | 1816±174 | 34% |
| Tecentriq+Avastin | 8 | 26.4±0.5 | 27.5±1.5 | 49±3 | 1848±178 | 32% |

| | | | | | | |
|---|---|---|---|---|---|---|
| Note: ^{a.} average ± standard errors. | | | | | | |

In a separate experiment using the same tumor model, the tumor inhibiting activity of TAF-6 was compared with that of the positive control M7824 analog. Mice were divided into 4 groups with 6 mice in each group. The groups were as follows: Vehicle group, TAF-6 (7 mg/kg, i.p., tiw × 8 times) group, M7824 analog (6 mg/kg, i.p., tiw × 8 times) group, and Avastin (5 mg/kg, i.p., tiw × 8 times) group. As shown in Fig. 6, the antitumor effect of TAF-6 was significantly better than that of the M7824 analog and Avastin at equimolar concentration doses.

### Embodiment 8 Antitumor activities of multi-domain fusion proteins in humanized mice transplanted with colon cancer HCT116 cells and PBMC

HCT-116 (human colon cancer) cells were used for modeling in immune system-humanized mice (NOG mouse) engrafted with human PBMC, so as to determine the *in vivo* efficacy of the multi-domain fusion proteins of the present disclosure. 7 to 9 weeks-old female NOG mice were inoculated with HCT-116 cells (3*10E6 + matrigel). PBMC (5*10E6/0.2ml) was injected into the mice through the tail vein 3 days later, then the tumor volume and body weight were observed. Mice with a tumor volume between 60-100 mm³ were selected and randomly divided into 6 groups based on their tumor volumes and body weights. The groups were as follows: Vehicle group, TAF-6 low-dose (2 mg/kg, i.p., tiw × 3) group, TAF-6 medium-dose (7 mg/kg, i.p., tiw × 3) group, TAF-6 high-dose (25 mg/kg, i.p., tiw × 3) group, Tecentriq (5 mg/kg, i. p., tiw× 3) group, and Tecentriq+Avastin (5+5 mg/kg, i. p., tiw× 3) group. Each group contained 8 mice, and the mice were administered on the day of grouping. Tumor volumes and body weights were measured twice a week, and the relationship between changes in body weights and tumor volumes in tumor-bearing mice was recorded in relation to the duration of drug administration. At the end of the experiment, the mice were euthanized, the tumors were weighed and photographed, and serum collection and tumor fixation were performed. The tumor growth inhibiting rate TGI_{TV} (%) was calculated and statistically analyzed.

Before grouping and at the end of the experiment, blood was collected from the orbital vein of mice. FACS analysis results showed the presence of human CD45-positive cells in the peripheral blood of mice in each group, and the proportion of CD45-positive cells increased over time, indicating successful humanization of the mouse immune systems.

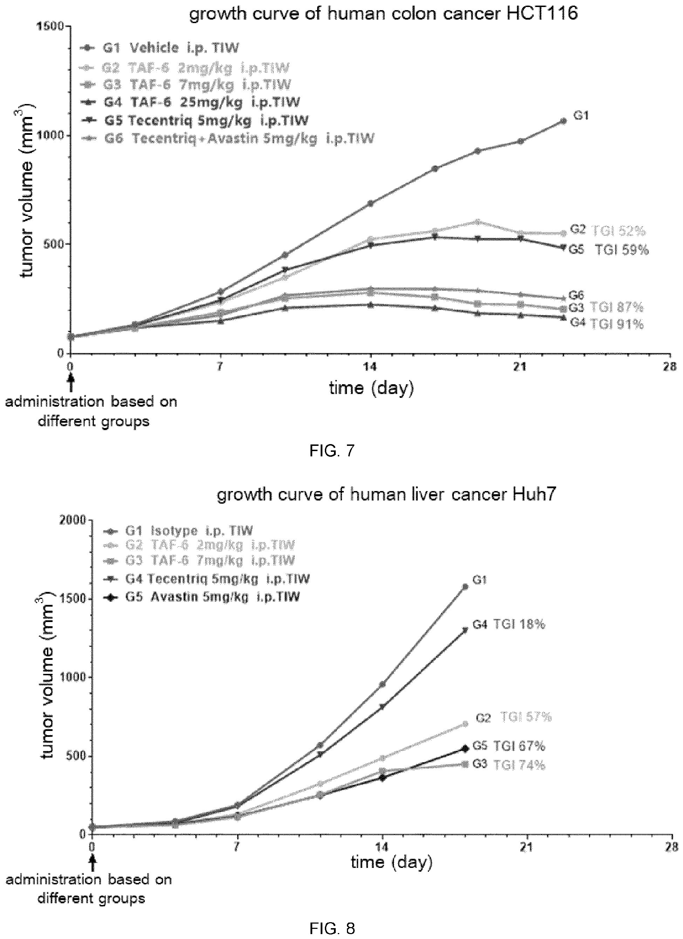

The results were shown in FIG. 7. In summary, the drug TAF-6 had a significant anti-tumor effect on the humanized mice subcutaneously transplanted with colon cancer HCT116 and PBMC, which effectively inhibited tumor growth. The anti-tumor effects of TAF-6 high-dose group and TAF-6 medium-dose group were significantly better than that of Tecentriq. At equimolar concentration dose, the efficacy of TAF-6 medium-dose (7 mg/kg, i.p., tiw × 3) group was still not inferior, even superior, to that of the Tecentriq+Avastin (5+5 mg/kg, i.p., tiw × 3) group.

### Embodiment 9 Antitumor activities of multi-domain fusion proteins in humanized mice engrafted with Huh-7 liver cancer cells and PBMC

PBMC cells were inoculated into mice, and human-derived Huh-7 liver cancer cells were inoculated subcutaneously to the right anterior rib of male NCG mice 45 days later. When the tumor size reached about 50 mm³, mice were divided into 5 groups with 10 mice in each group. The groups were as follows: Isotype group, TAF-6 low-dose (2 mg/kg, i.p., tiw × 8) group, TAF-6 high-dose (7 mg/kg, i.p., tiw × 8) group, Tecentriq (5 mg/kg, i.p., tiw × 8) group, and Avastin (5 mg/kg, i.p., tiw × 8) group, and administration was performed. Tumor volume and body weight were measured twice a week, and the relationship between changes in body weights and tumor volumes in tumor-bearing mice was recorded in relation to the duration of drug administration. At the end of the experiment, the mice were euthanized, the tumors were weighed and photographed, and serum collection and tumor fixation were performed. The tumor growth inhibiting rate TGI_{TV} (%) was calculated and statistically analyzed.

Before grouping and at the end of the experiment, blood was collected from the orbital vein of mice. FACS analysis results showed the presence of human CD45-positive cells in the peripheral blood of mice in each group, and the proportion of CD45-positive cells increased over time, indicating successful humanization of the mouse immune systems.

During the treatment period, the mice in each group were fed and watered normally, and their body weights were generally stable.

At the end of the experiment (PG-D18), the tumor growth inhibiting rates of TAF-6 low-dose group, TAF-6 high-dose group, Tecentriq group, and Avastin group were 57%, 74%, 18% and 67%, respectively. Except for Tecentriq group, the tumor volumes of TAF-6 low-dose group, TAF-6 high-dose group, and Avastin group were significantly smaller than that of Isotype group (all p values were less than 0.01), the tumor volume of TAF-6 high-dose group was significantly smaller than that of Tecentriq group (p<0.01), and the tumor volume in Avastin group was significantly smaller than that in Tecentriq group (p<0.05). The results were shown in FIG. 8.

In summary, TAF-6 showed a significant anti-tumor effect on the humanized mice subcutaneously transplanted with Huh-7 liver cancer cells and PBMC, which effectively inhibited tumor growth. The anti-tumor effect of TAF-6 was significantly better than that of Tecentriq, and the tumor inhibiting effect of TAF-6 was enhanced with the increase in dose.

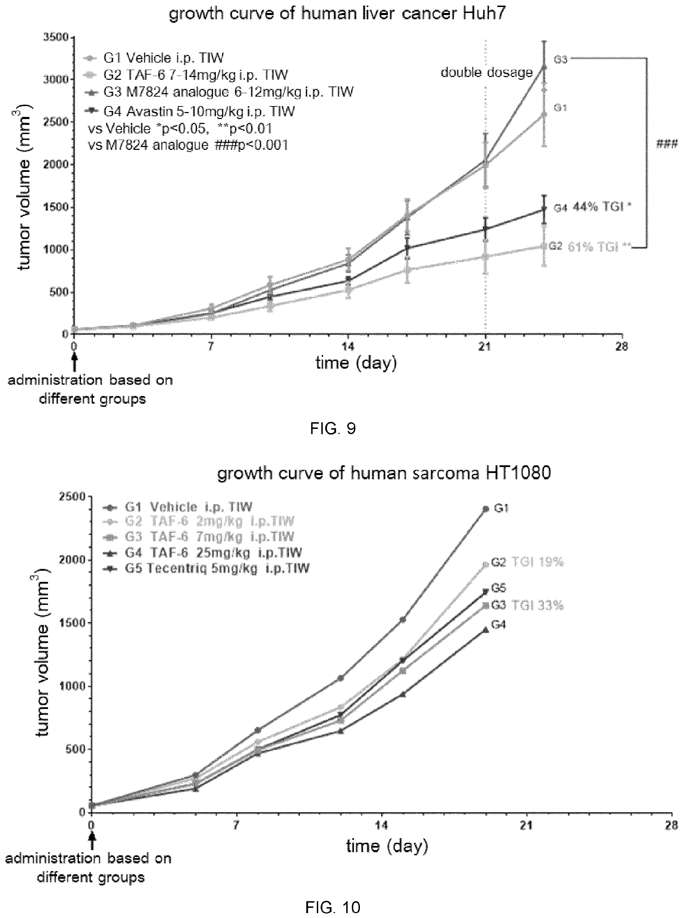

In a separate experiment using the same tumor model, the tumor inhibiting activity of TAF-6 was compared with that of the positive control M7824 analog. Mice were divided into 4 groups with 6 mice in each group. The groups were as follows: Vehicle group, TAF6 (7 mg/kg, i.p., tiw x 9; later the dose was adjusted to 14 mg/kg, i.p., tiw × 2) group, M7824 analog (6 mg/kg, i.p., tiw × 9; later the dose was adjusted to 12 mg/kg, i.p., tiw × 2) group, and Avastin (5 mg/kg, i.p., tiw × 9; later the dose was adjusted to 10 mg/kg, i.p., tiw × 2) group. As shown in Fig. 9, the antitumor effect of TAF-6 was significantly better than that of the M7824 analog.

### Embodiment 10 Antitumor activities of multi-domain fusion proteins in humanized mice transplanted with HT1080 sarcoma cells and PBMC

PBMC cells were inoculated into mice, and human-derived HT1080 sarcoma cells were inoculated subcutaneously to the right anterior rib of male NCG mice seven days later. When the tumor size reached about 56 mm³, mice were divided into 5 groups with 8 mice in each group, which were Vehicle group, TAF-6 low-dose (2 mg/kg, i.p., tiw × 9) group, TAF-6 medium-dose (7 mg/kg, i.p., tiw × 9) group, TAF-6 high-dose (25 mg/kg, i.p., tiw × 9) group, and Tecentriq (5 mg/kg, i.p., tiw × 9) group, and administration was performed. Tumor volume and body weight were measured every week, and the relationship between changes in body weights and tumor volumes in tumor-bearing mice was recorded in relation to the duration of drug administration.

At the end of the experiment, the mice were euthanized, the tumors were weighed and photographed, and serum collection and tumor fixation were performed. The tumor growth inhibiting rate TGI_{TV} (%) was calculated and statistically analyzed.

Before grouping and at the end of the experiment, blood was collected from the orbital vein of mice. FACS analysis results showed the presence of human CD45-positive cells in the peripheral blood of mice in each group, and the proportion of CD45-positive cells increased over time, indicating successful humanization of the mouse immune systems.

At the end of the experiment (PG-D19), the tumor growth inhibiting rates of TAF-6 low-dose group, TAF-6 medium-dose group, TAF-6 high-dose group, and Tecentriq group were 19%, 33%, 41%, and 28%, respectively. The tumor volumes of TAF-6 medium-dose group and Tecentriq group were significantly smaller than that of Vehicle group (all p values were less than 0.05), and there was no significant difference in tumor volumes among the groups (p>0.05). The results were shown in FIG. 10.

In summary, the present disclosure effectively overcomes the shortcomings in the prior art and has a high industrial utilization value.

The above embodiments of the present disclosure are only examples for clearly illustrating the present disclosure, and are not intended to limit the implementations of the present disclosure. Any person skilled in the art can modify or change the above embodiments without departing from the spirit and scope of the present disclosure. Therefore, all equivalent modifications or changes made by those who have common knowledge in the technical field without departing from the spirit and technical ideas disclosed by the present disclosure should still be covered by the claims of the present disclosure.

## Claims

1. A fusion protein, comprising an anti-PD-L1 single domain antibody fragment, an anti-VEGF fragment, and a TGF-β binding fragment.

2. The fusion protein according to claim 1, wherein complementary determining regions of the anti-PD-L1 single domain antibody fragment comprise CDR1, CDR2, and CDR3,
wherein CDR1 has an amino acid sequence as shown in one of SEQ ID NOs: 1-5, CDR2 has an amino acid sequence as shown in one of SEQ ID NOs: 6-9, and CDR3 has an amino acid sequence as shown in one of SEQ ID NOs: 10-15.

3. The fusion protein according to claim 2, wherein the complementary determining regions of the anti-PD-L1 single domain antibody fragment comprise CDR1-CDR3, wherein
CDR1 has an amino acid sequence of SEQ ID NO: 1, CDR2 has an amino acid sequence of SEQ ID NO: 6, and CDR3 has an amino acid sequence of SEQ ID NO: 10;
CDR1 has an amino acid sequence of SEQ ID NO: 2, CDR2 has an amino acid sequence of SEQ ID NO: 7, and CDR3 has an amino acid sequence of SEQ ID NO: 11;
CDR1 has an amino acid sequence of SEQ ID NO: 3, CDR2 has an amino acid sequence of SEQ ID NO: 7, and CDR3 has an amino acid sequence of SEQ ID NO: 12;
CDR1 has an amino acid sequence of SEQ ID NO: 4, CDR2 has an amino acid sequence of SEQ ID NO: 8, and CDR3 has an amino acid sequence of SEQ ID NO: 13;
CDR1 has an amino acid sequence of SEQ ID NO: 2, CDR2 has an amino acid sequence of SEQ ID NO: 7, and CDR3 has an amino acid sequence of SEQ ID NO: 14; or
CDR1 has an amino acid sequence of SEQ ID NO: 5, CDR2 has an amino acid sequence of SEQ ID NO: 9, and CDR3 has an amino acid sequence of SEQ ID NO: 15.

4. The fusion protein according to claim 2, wherein the anti-PD-L1 single domain antibody fragment further comprises framework regions, wherein the framework regions comprise FR1, FR2, FR3, and FR4, wherein
FR1 has an amino acid sequence of SEQ ID NO: 49, FR2 has an amino acid sequence as shown in one of SEQ ID NOs: 50-52, and FR3 has an amino acid sequence as shown in one of SEQ ID NOs: 53-55, and FR4 has an amino acid sequence of SEQ ID NO: 56.

5. The fusion protein according to claim 4, wherein the framework regions comprise FR1-FR4, wherein
FR1 has an amino acid sequence of SEQ ID NO: 49, FR2 has an amino acid sequence of SEQ ID NO: 50, FR3 has an amino acid sequence of SEQ ID NO: 53, and FR4 has an amino acid sequence of SEQ ID NO: 56;
FR1 has an amino acid sequence of SEQ ID NO: 49, FR2 has an amino acid sequence of SEQ ID NO: 51, FR3 has an amino acid sequence of SEQ ID NO: 54, and FR4 has an amino acid sequence of SEQ ID NO: 56;
FR1 has an amino acid sequence of SEQ ID NO: 49, FR2 has an amino acid sequence of SEQ ID NO: 52, FR3 has an amino acid sequence of SEQ ID NO: 54, and FR4 has an amino acid sequence of SEQ ID NO: 56; or
FR1 has an amino acid sequence of SEQ ID NO: 49, FR2 has an amino acid sequence of SEQ ID NO: 52, FR3 has an amino acid sequence of SEQ ID NO: 55, and FR4 has an amino acid sequence of SEQ ID NO: 56.

6. The fusion protein according to claim 2, wherein the anti-PD-L1 single domain antibody fragment comprises:
a) a polypeptide fragment having an amino acid sequence as shown in one of SEQ ID NOs:16-21; or
b) a peptide fragment having an amino acid sequence sharing at least 90% sequence identity with one of SEQ ID Nos: 16-21 and a function of the polypeptide fragment in a);
and/or, the anti-PD-L1 single domain antibody fragment is derived from Vicugna pacos;
and/or, the anti-PD-L1 single domain antibody fragment is humanized.

7. The fusion protein according to claim 1, wherein the anti-VEGF fragment is a Bevacizumab,
preferably, the anti-VEGF fragment comprises:
c) a polypeptide fragment having an amino acid sequence as shown in one of SEQ ID Nos:22-23; or
d) a peptide fragment having an amino acid sequence sharing at least 90% sequence identity with one of SEQ ID Nos: 22-23 and a function of the polypeptide fragment in c);
and/or, the anti-PD-L1 single domain antibody fragment is derived from Mus musculus;
and/or, the anti-PD-L1 single domain antibody fragment is humanized.

8. The fusion protein according to claim 1, wherein the TGF-β binding fragment is a TGF-β RII extracellular region structural fragment,
preferably, the TGF-β binding fragment comprises:
e) a polypeptide fragment having an amino acid sequence of SEQ ID No: 24; or
f) a peptide fragment having an amino acid sequence sharing at least 90% sequence identity with SEQ ID No: 24 and a function of the polypeptide fragment in e);
and/or, the TGF-β binding fragment is derived from homo sapiens.

9. The fusion protein according to claim 1, wherein the fusion protein further comprises a linker peptide fragment,
preferably, the linker peptide fragment is rich in G, S, and/or A,
more preferably, the linker peptide fragment is a flexible polypeptide chain consisting of G glycine and/or S serine and/or A alanine,
wherein a length of the amino acid sequence of the linker peptide fragment is 3-30.

10. The fusion protein according to claim 9, wherein the linker peptide fragment comprises a polypeptide fragment having an amino acid sequence as shown in one of SEQ ID Nos: 34-36;
and/or, a first linker peptide fragment is provided between the anti-PD-L1 single domain antibody fragment and the anti-VEGF fragment;
and/or, a second linker peptide fragment is provided between the anti-VEGF fragment and the TGF-β binding fragment.

11. The fusion protein according to any one of claims 1-10, wherein the fusion protein sequentially comprises the anti-PD-L1 single domain antibody fragment, the anti-VEGF fragment, and the TGF-β binding fragment from N-terminus to C-terminus;
and/or, the anti-PD-L1 single domain antibody fragment is located at N-terminus of a heavy chain of the anti-VEGF fragment;
and/or, the anti-PD-L1 single domain antibody fragment is located at N-terminus of a light chain of the anti-VEGF fragment;
and/or, the TGF-β binding fragment is located at C-terminus of the heavy chain of the anti-VEGF fragment.

12. The fusion protein according to claim 1, wherein the fusion protein comprises an amino acid sequence comprising one of SEQ ID NO: 23, and SEQ ID NOs: 25-33;
or, the fusion protein comprises an amino acid sequence comprising SEQ ID NO: 25 and SEQ ID NO: 26;
the fusion protein comprises an amino acid sequence comprising SEQ ID NO: 25 and SEQ ID NO: 27;
the fusion protein comprises an amino acid sequence comprising SEQ ID NO: 25 and SEQ ID NO: 28;
the fusion protein comprises an amino acid sequence comprising SEQ ID NO: 25 and SEQ ID NO: 29;
the fusion protein comprises an amino acid sequence comprising SEQ ID NO: 30 and SEQ ID NO: 27;
the fusion protein comprises an amino acid sequence comprising SEQ ID NO: 30 and SEQ ID NO: 29;
the fusion protein comprises an amino acid sequence comprising SEQ ID NO: 31 and SEQ ID NO: 23;
the fusion protein comprises an amino acid sequence comprising SEQ ID NO: 32 and SEQ ID NO: 23; or
the fusion protein comprises an amino acid sequence comprising SEQ ID NO: 33 and SEQ ID NO: 23.

13. An isolated polynucleotide, encoding the fusion protein according to any one of claims 1-12.

14. A construct, comprising the isolated polynucleotide according to claim 13.

15. An expression system, comprising the construct according to claim 14 or the isolated polynucleotide according to claim 13 which is incorporated into a genome.

16. A method for preparing the fusion protein according to any one of claims 1-12, comprising:
culturing the expression system according to claim 15 under a condition suitable for expressing a fusion protein, and
performing isolation and purification to provide the fusion protein.

17. Use of the fusion protein according to any one of claims 1-12, or a culture of the expression system according to claim 15 in the preparation of medications.

18. The use according to claim 17, wherein the medication is used for treating tumors; preferably, the tumor is selected from a group consisting of lung cancer, melanoma, gastric cancer, ovarian cancer, colon cancer, liver cancer, renal cancer, bladder cancer, breast cancer, classical Hodgkin's lymphoma, hematological malignancy, sarcoma, head and neck cancer, and nasopharyngeal cancer.

19. A pharmaceutical composition, comprising the fusion protein according to any one of claims 1-12 or a culture of the expression system according to claim 15.
